# EUROPEAN PATENT SPECIFICATION

(11) **EP 1 303 582 B1**
(45) Date of publication and mention of the grant of the patent: **23.09.2009**
(21) Application number: 01955906.1
(22) Date of filing: 20.07.2001
(51) Int. Cl.: C11D 3/386, C12N 9/96, C12N 9/54

(54) **STABILIZATION OF ENZYMES**
ENZYMSTABILISIERUNG
STABILISATION D'ENZYMES

(30) Priority: 22.07.2000 US 220208 P
(43) Date of publication of application: 23.04.2003
(73) Proprietor: GENENCOR INTERNATIONAL, INC., Palo Alto, California 94304 (US)
(72) Inventor: BECKER, Nathaniel, T., Hillsborough, CA 94010 (US); BOTT, Richard, T., Burlingame, CA 94010 (US); BOWDEN, Shauna, L., Mountain View, CA 94040 (US); HENG, Meng, Hong, Belmont, CA 94002 (US); PAECH, Christian, Daly City, CA 94014 (US); KOSOLA, Antti, V., FIN-02400 Kirkkonummi (FI)
(74) Representative: Wibbelmann, Jobst
(86) International application number: PCT/US2001/023076
(87) International publication number: WO 2002/008398

(56) References cited:
- WO-A-00/12663
- GB-A- 1 232 627
- US-A- 3 296 094
- US-A- 4 169 817
- US-A- 5 269 960
- US-A- 5 759 984

## Description

### BACKGROUND OF THE INVENTION

### 1. Field of the Invention

The present invention relates to methods for stabilizing one or more protease enzymes from Bacillus species in a liquid medium and to stabilized liquid enzyme formulations with the stabilized protease enzymes.

### 2. Background

The stabilization of enzymatic activity is a standing problem in all areas of technology where enzymes are likely to be applied. Stability in this sense includes resistance to decrease in enzymatic activity prior to usage, e.g., under storage conditions. Stabilization of enzymes in liquid formulations is particularly a problem. For example, a pre-formulated liquid enzyme concentrate may sometimes be stored for weeks or months before eventually being blended into a final product (e.g., a personal care product, such as a hand cream; or a cleaning product, such as a liquid detergent). Similarly, formulated liquid products containing enzymes may sit in storage for lengthy periods of time before use, as well. For a variety of reasons, the activity of enzymes in liquid formulations typically decreases over time.

The prior art has attempted to deal with this problem. For example, organic compounds, such as sodium formate, and propylene glycol or glycerol are often added to liquid enzyme formulations. U.S. Pat. No. 3,296,094, Theodore Cayle, issued January 3, 1967, discloses aqueous solutions of a proteolytic enzyme, in particular papain, which are stabilized against loss of proteolytic activity upon storage by incorporating a combination of glycerol and partially hydrolyzed and solubilized collagen. WO 00/12663 A describes a stable liquid enzyme composition for cleaning contact lenses comprising an enzyme, such as subtilisin, one or more polyol(s), a borate/boric acid compound, calcium ions and water.U.S. Pat. No. 4,318,818, Letton et al., issued March 9, 1982, discloses liquid detergents containing enzymes and an enzyme-stabilizing system comprising calcium ions and a low molecular weight carboxylic acid or salt, preferably a formate. The composition preferably contains an anionic surfactant and a saturated fatty acid. U.S. Pat. No. 4,404,115, Tai, issued September 13, 1983, discloses cleaning compositions containing enzymes, alkali metal pentaborate, alkaki metal sulfite and a polyol. While somewhat effective, additives such as formate and other organic salts unfortunately are costly and, thus, significantly add to the expense of the liquid enzyme concentrate and final product. Further, there is in some instances a desire for formulations which are acceptable for food, pharmaceutical, or cosmetic use, and certain salts like sodium formate may not be acceptable or suitable for this purpose.

The use of inorganic salts as stabilization agents for enzymes is known in the art. For instance, U.S. Pat. No. 5,460,658, Nakagawa et al, issued October 24,1995, discloses an enzyme based contact lens cleaning solution said to be stabilized with a polyhydric alcohol (5-30%) and alkali metal salt (1-5%) combination. The Nakagawa et al. patent teaches that polyhydric alcohol levels are less than 30% to maintain enzyme activity in the contact lens solution application. Xylanase was stabilized with a polyol (20-40%) and formate or potassium chloride (4-8%) as described in a study entitled "Development of A Method for the Stabilization and Formulation of Xylanase from Trichoderma Using Experimental Design", R Spencer Fisk and Curran Simpson, as reported in Studies in Organic Chemistry, vol. 47, Stability and Stabilization of Enzymes, Elservier, edited by W.J.J. Van Den Tweel, A. Harder, R.M. Buitelaar, 1992. This study discloses the use of no more than 40% polyol to maintain xylanase activity.

A cosmetic formulation having an enzyme stabilized using a 30-99% water-binding polyol that is partially or totally complexed with acrylic or methacrylic polymer is described in U.S. Pat. No. 5,830,449, Afriat et al., issued November 3, 1998 , and U.S. Pat. No. 5,703,041, Afriat et al., issued December 30, 1997. The cosmetic formulation may also include an inorganic salt (2 to 12%) as a secondary stabilizing agent.

Clearly, there is a continuing need for liquid formulations that contain enzymes which are stabilized and exhibit a high activity over time. Particularly, there is a need for protease stabilized liquid formulations that are easy to process, highly effective, inexpensive to produce relative to the previously used stabilizing formulations, relatively inactive until ultimately included as an ingredient in a selected application, and also useful for formulations that are well tolerated physiologically.

### SUMMARY OF THE INVENTION

It has been discovered that the combination of a high level of alkali metal halide salts, such as sodium chloride (at least 4% or 5% w/w, or higher), in combination with a polyol solvent, such as glycerol, propylene glycol, sucrose, etc (30% w/w, or higher), provides a high level of thermal stability for protease (produced from Bacillus species, e.g., subtilisin) formulations.

The present invention provides a method for stabilizing one or more protease enzymes from Bacillus species in a liquid medium. In one embodiment, the method of the invention consists of formulating in the liquid medium a high level of an alkali metal halide salt in combination with a polyol solvent.

The alkali metal halide salt can be, for example, sodium chloride, potassium chloride, and sodium or potassium fluoride or bromide. Lithium salts may also be used. Additionally, other inorganic salts, such as salts of sulfates or sulfites, carbonates, phosphates, silicates or nitrates may be used. One preferred embodiment contemplates at least between 4-12% (e.g., at least 4%, at least 5%, at least 6%, at least 7%, at least 8%, at least 9%, at least 10%, at least 11%, and/or at least 12%) sodium chloride. In one embodiment, the sodium chloride comprises between about 5-12%, preferably about 8 -12% w/w, of the liquid formulation.

The polyol can be present, for example, at a level of at least 30%, at least 40%, at least 50%, at least 60%, at least 70%, at least 80%, at least 90%, and/or at least 95%. For example, one embodiment contemplates between 50 to 98% polyol, preferably between 55 and 95% polyol, preferably between 80 to 90% polyol, and most preferably 50 to 80% polyol. Suitable polyols include, for example, glycerol, propylene glycol, sucrose, among others.

Among the protease enzymes from Bacillus species that can be stabilized by the teachings herein, one preferred enzyme is a subtilisin.

The liquid medium of the invention can be, for example, a liquid enzyme concentrate or a formulated product including at least one protease. In one embodiment, the liquid medium is a formulated product selected from the group consisting of personal care products, health care products, and cleaning/detergency products. The liquid medium can be used, for example, in formulating hand creams, liquid detergents, and the like.

The present invention further provides a liquid formulation providing an enzyme-stabilizing environment. In one embodiment, the formulation includes one or more proteases from a Bacillus species, a high level of an alkali metal salt, and a polyol solvent.

The alkali metal halide salt can be, for example, sodium chloride, potassium chloride, and sodium or potassium fluoride or bromide. Lithium salts also may be used. Additionally, other inorganic salts, such as salts of sulfates or sulfites, carbonates, phosphates, silicates or nitrates may be used. In one embodiment, the sodium chloride is present at a level of between 4-12% (e.g., at least 4%, at least 5%, at least 6%, at least 7%, at least 8%, at least 9%, at least 10%, at least 11%, up to and including 12%). In an exemplary formulation, the sodium chloride comprises about 8-12% w/w of the liquid formulation.

The polyol is preferably present, for example, at a level of at least 30%. Certain embodiments contemplate a polyol level of at least 30%, at least 40%, at least 50%, at least 60%, at least 70%, at least 80%, at least 90%, and/or at least 95%. One embodiment contemplates between 50 to 98% polyol, preferably between 55 and 95% polyol, preferably between 80 to 90% polyol, and most preferably 50 to 80 polyol. Suitable polyols include, for example, glycerol, propylene glycol, sucrose, among others.

Among the one or more proteases from a Bacillus species that are stabilized in the formulation, one preferred embodiment contemplates inclusion of a subtilisin.

In one embodiment, the liquid formulation is a liquid enzyme concentrate or a formulated product including at least one protease produced from Baccillus species, such as a subtilisin. The liquid formulation can be, for example, a formulated product such as a personal care product, health care product, a cleaning product, a detergency product, among others.

The present invention also provides a stabilized liquid enzyme formulation, including: (i) a protease from Baccillus species, such as subtilisin; (ii) at least 5% w/w of an alkali metal halide salt, such as sodium chloride, potassium chloride, sodium or potassium fluoride or bromide, or lithium salts. Additionally, other inorganic salts, such as salts of sulfates or sulfites, carbonates, phosphates, silicates or nitrates may be used; and (iii) at least 30% w/w of a polyol solvent, such as glycol. Preferably, the formulation includes a subtilisin which is stabilized to exhibit at least about 98% activity remaining after 22 days at 37 degrees C. In one embodiment, the stabilized subtilisin includes an amino acid substitution at position 217 (e.g., Y217L).

These and other features, aspects and advantages of the present invention will become apparent from the following detailed description, in conjunction with the appended claims.

### DETAILED DESCRIPTION OF THE INVENTION

The present invention provides methods for stabilizing protease enzymes from Baccillus species and liquid formulations including the stabilized enzymes utilizing a polyol and an alkali metal halide salt.

Percentages herein are expressed as a percentage by weight of the liquid formulation.

The inventors hereof have determined that the combination of a high level of an alkali metal halide salt (e.g., sodium chloride) in combination with a polyol solvent (e.g., glycol) provides a high level of thermal stability for liquid enzyme (e.g., proteases produced from Bacillus species) formulations.

Formulations of the present invention include at least about 30% polyol or polyhydric alcohol; for example between from about 50 to about 98%, between from about 50 to about 95%, between from about 80 to about 90% polyol, and/or between from about 50-80% polyol. An exemplary liquid formulation suitable for inclusion in personal care applications, as contemplated herein, includes between from about 50% to about 80% glycerol. Other suitable polyols include, and are not limited to, propylene glycol, ethylene glycol, sorbitol, mannitol, erythritol, dulcitol and inositol. Such a liquid formulation can be further formulated into, for example, a hand cream, or the like. A liquid formulation for liquid detergent applications, as contemplated herein, includes between from about 33 to about 40% propylene glycol. Another exemplary liquid formulation for personal care application includes about 60% glycerol. The polyol level is approximately 50-80% to produce a compound having relatively inactive enzymes until such compound ultimately is included in a specific application.

Formulations of the present invention include at least about 5% of an alkali metal halide salt, such as sodium chloride, potassium chloride, and sodium or potassium fluoride or bromide, or lithium salts. Additionally, other inorganic salts, such as salts of sulfates or sulfites, carbonates, phosphates, silicates or nitrates may be used. In one preferred embodiment, the alkali halide metal salt is sodium chloride. The sodium chloride can be present, for example, at a level of at least about 5%, at least about 6%, at least about 7%, at least about 8%, at least about 9%, at least about 10%, at least about 11%, and/or at least about 12%. In one embodiment, a liquid enzyme formulation includes between from about 5% to about 12% sodium chloride (e.g., preferred at about 8% sodium chloride). In another preferred embodiment, the liquid enzyme formulation includes about 10% sodium chloride. Enzymes for use in the present invention include proteases a produced from any Bacillus species and include mutants which retain their bacillus protease-like structure and function.

Preferred proteases from Bacillus species include, for example, Purafect and Properase, from Genencor International, Inc. or Savinase, Esperase, Alcalase available from Novozymes A/S. One or more enzymes may be included in the formulations of the present invention.

The stabilized liquid enzyme formulations of the present invention can be applied in a variety of fields, including the fields of personal care (e.g., protease for use in hand creams, and the like), and cleaning (e.g., protease for use in liquid detergents, and the like), among others. For example, the liquid enzyme formulations can be liquid enzyme concentrates which are useful for further formulation into final products, and/or they can be final formulated products, such as skin creams, lotions, liquid soaps, liquid detergents, etc.

Liquid enzyme formulations of the present invention are particularly effective at stabilizing enzymes during storage, within a temperature range of between from about 20 to about 40 degrees Celsius, e.g., at or around room temperature (22-25 degrees Celsius).

### EXAMPLES

The following examples are illustrative and are not intended to limit the invention.

### Example 1

Subtilisin BPN' Y217L was formulated at 5-7 gE/l in a base of 80-90% glycerol, 1 mM KH2PO4 at pH 5.0. The method of formulation generally involves the following: the 1mM CaCl₂ reagent was added to the glycerol reagent followed by the addition of the selected enzyme. Water was then added and the resulting mixture was allowed to dissolve overnight at 4° C. The selected salt concentration was prepared by dissolving the salt in 1M NaOAC prior to addition to the dissolved enzyme. Next, 3% CP carbon was added to the enzyme salt combination and the resulting compound was mixed at 22° C for approximately 6 hours. Next, the compound was filtered and the resulting solution was stored for the stability studies set out below.

As shown in Table 1, the formulation was tested without further stabilization and with stabilization utilizing three stabilizers (sodium acetate, sodium chloride, and sodium formate) .

**TABLE 1: Stability Study 1**

| | | | | |
|---|---|---|---|---|
| **Basic Conditions for all samples** | | | | |
| * 50 ppm Ca2+ 10 g enzyme solution | | | | |
| * Temperature 25C/37C | | | | |

| **Experiments** | | | | **Product Activity** |
|---|---|---|---|---|
| B0 | KH2PO4 10 mM @ pH 5.0 before additions; 10% | **Sodium Acetate; 8%** | Glycerol; 82% | 6.75 gE/l |
| B1 | KH2PO4 10 mM @ pH 5.0 before additions; 10% | **Sodium Chloride; 8%** | Glycerol; 82% | 6.75 gE/l |
| B2 | KH2PO4 10 mM @ pH 5.0 before additions; 10% | **Sodium Formate; 8%** | Glycerol; 82% | 6.75 gE/l |
| B3 | KH2PO4 10 mM @ pH 5.0 before additions; 10% | None | Glycerol; 90% | 6.75 gE/l |
| B4 | KH2PO4 10 mM @ pH 5.0 before additions; 10% | None | Glycerol; 90% | 3.37 gE/l |
| B5 | KH2PO4 10 mM @ pH 5.0 before additions; 10% | None | Glycerol; 45% / **Propylene Glycol; 1345%** | 6.75 gE/l |
| B6 | KH2PO4 10 mM @ pH 5.0 before additions; 10% | None | **Propylene Glycol; 90%** | 6.75 gE/l |
| B7 | KH2PO4 10 mM @ **pH 5.0 after** additions; 10% | None | Glycerol; 90% | 6.75 gE/l |
| B8 | KH2PO4 10 mM @ **pH 5.5** before additions; 10 % | None | Glycerol; 90% | 6.75 gE/l |

Activity levels for all of the above formulations were monitored as exhibited by the data of Table 2, which demonstrates that sodium chloride is a very effective agent for stabilization of subtilisin (note, in particular, B1). Remaining enzyme activity at 22 days utilizing the three stabilizers as shown was between 96-98% as compared to a value of only 81 % in the remaining formulations that did not include sodium acetate, sodium chloride or sodium formate.

**Table 2: 37 °C**

| | | | | | | | | | |
|---|---|---|---|---|---|---|---|---|---|
| Subtilisin Stability Study | | | | | | | | | |
| Activities by AAPF-pNA Assay in ΔAU min⁻¹ml⁻¹ | | | | | | | | | |

| | Buffer | | | | | | | | |
|---|---|---|---|---|---|---|---|---|---|
| Day | 0 | 1 | 2 | 3 | 4 | 5 | 6 | 7 | 8 |
| 0 | 206 | 211 | 233 | 229 | 112 | 234 | 204 | 200 | 204 |
| 1 | 212 | 216 | 239 | 238 | 113 | 238 | 214 | 171 | 214 |
| 6 | 205 | 210 | 228 | 214 | 98 | 217 | 177 | 161 | 198 |
| 22 | 196 | 206 | 224 | 185 | 49 | 179 | 109 | 142 | 160 |
| 41 | 186 | 208 | 212 | 150 | 11 | 163 | 62 | 116 | 138 |
| 66 | 186 | 205 | 202 | 129 | 7 | 132 | 32 | 33 | 113 |
| 94 | 183 | 192 | 183 | | | | | | 88 |

| | Activity left % Buffer | | | | | | | | |
|---|---|---|---|---|---|---|---|---|---|
| Day | B0 | B1 | B2 | B3 | B4 | B5 | B6 | B7 | B8 |
| 0 | 100 | 100 | 100 | 100 | 100 | 100 | 100 | 100 | 100 |
| 1 | 103 | 102 | 102 | 104 | 101 | 102 | 105 | 85 | 105 |
| 6 | 100 | 100 | 98 | 94 | 88 | 93 | 87 | 80 | 97 |
| 22 | 96 | 98 | 96 | 81 | 43 | 77 | 54 | 71 | 78 |
| 41 | 91 | 99 | 91 | 66 | 10 | 70 | 30 | 58 | 67 |
| 66 | 91 | 97 | 87 | 57 | 6 | 57 | 16 | 16 | 55 |
| 94 | 89 | 91 | 79 | | | | | | 43 |

The 8% NaCl sample was found to have high stability (over 98 % activity remaining after 22 days at 37 degrees C and 100% after 23 days at 37 degrees C.). The data further demonstrates that a high activity level (at least 90%) remained in the presence of sodium chloride out to 94 days.

Table 3 below represents the same example as above conducted at 25° C.

**Table 3: 25 °C**

| | | | | | | | | | |
|---|---|---|---|---|---|---|---|---|---|
| Subtilisin Stability Study | | | | | | | | | |
| Activities by AAPF-pNA Assay in ΔAU min⁻¹ml⁻¹ | | | | | | | | | |

| | Buffer | | | | | | | | |
|---|---|---|---|---|---|---|---|---|---|
| Day | 0 | 1 | 2 | 3 | 4 | 5 | 6 | 7 | 8 |
| 0 | 206 | 211 | 233 | 229 | 112 | 234 | 204 | 200 | 204 |
| 2 | 205 | 216 | 223 | 227 | 108 | 227 | 200 | 184 | 197 |
| 23 | 204 | 212 | 242 | 214 | 94 | 223 | 188 | 166 | 203 |
| 30 | 200 | 213 | 220 | 214 | 85 | 215 | 182 | 153 | 197 |
| 65 | 201 | 206 | 227 | 192 | 40 | 187 | 158 | 135 | 174 |
| 94 | 189 | 197 | 222 | | | | | | 158 |

| | Activity left % Buffer | | | | | | | | |
|---|---|---|---|---|---|---|---|---|---|
| Day | B0 | B1 | B2 | B3 | B4 | B5 | B6 | B7 | B8 |
| 0 | 100 | 100 | 100 | 100 | 100 | 100 | 100 | 100 | 100 |
| 2 | 100 | 102 | 96 | 99 | 97 | 97 | 98 | 92 | 96 |
| 23 | 99 | 101 | 104 | 93 | 84 | 95 | 93 | 83 | 99 |
| 30 | 97 | 101 | 94 | 93 | 76 | 92 | 90 | 77 | 97 |
| 65 | 98 | 98 | 97 | 84 | 35 | 80 | 78 | 68 | 77 |
| 94 | 92 | 93 | 95 | | | | | | 77 |

Example 2 illustrates the results of a study designed to increase the commercial desirability of liquid enzyme formulations and to facilitate manufacturing of such formulations. Accordingly, Example 2 illustrates the surprising results achieved by reducing the concentration of glycerol and increasing the concentration of salt in the formulation.

Subtilisin BPN' Y217L was formulated as set out in Table 4 below in a base of 70-82% glycerol, 1 mM KH2PO4.

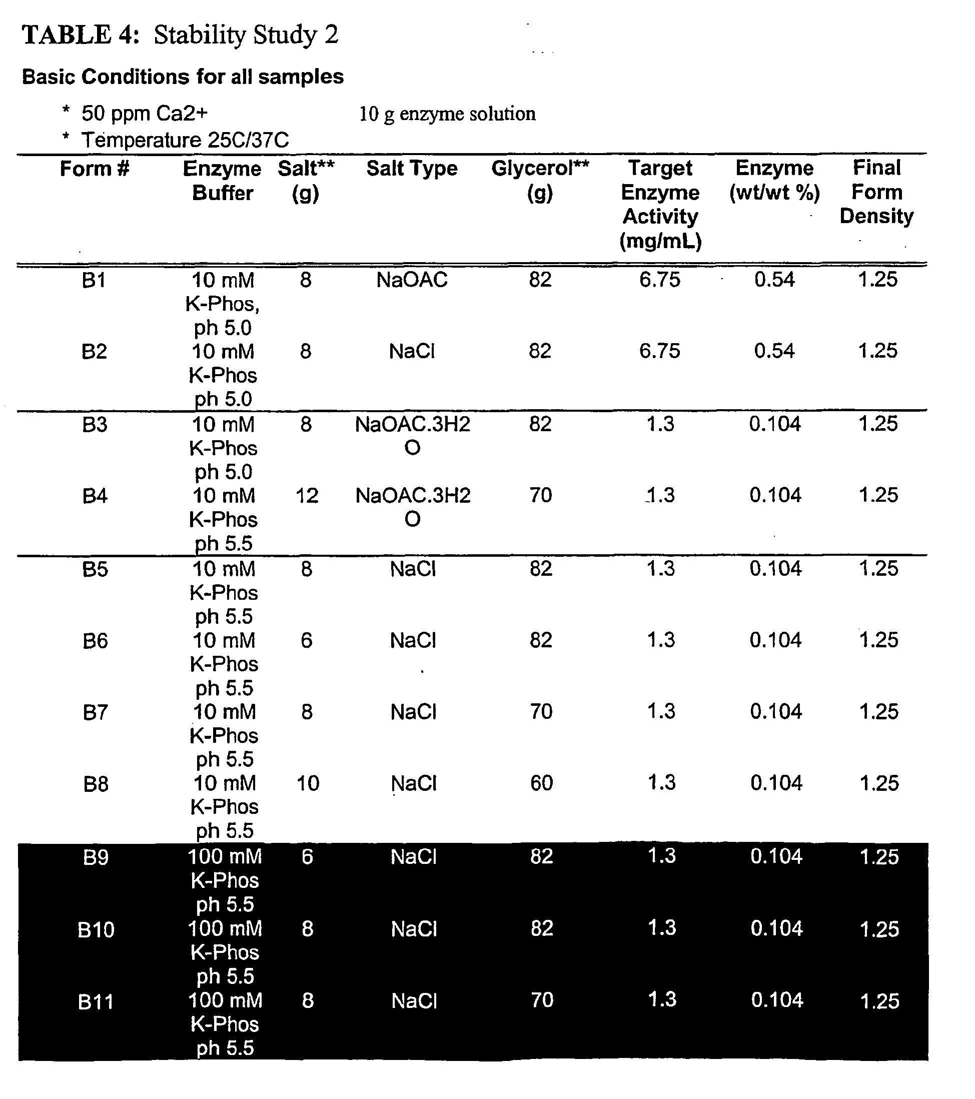

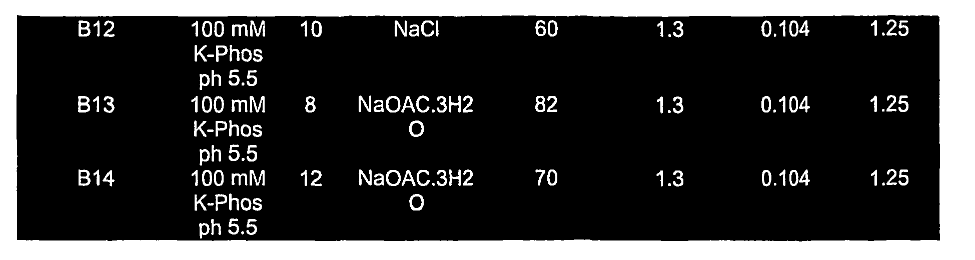

As shown by the surprising data of Table 5 below, a stable glycerol/salt formulation that is easily manufactured may be achieved by increasing the salt concentration thereby enabling a reduction in the amount of glycerol in the formulation. Table 5 illustrates that a 60% glycerol and 10% salt formulation is exceptionally stable.

**TABLE 5 Subtilisin Stability Study: 25°C Activities by AAPF-pNA Assay in ΔAU min⁻¹ml⁻¹**

| | Buffer | | | | | | | | | | | | | |
|---|---|---|---|---|---|---|---|---|---|---|---|---|---|---|
| Day | 1 | 2 | 3 | 4 | 5 | 6 | 7 | 8 | 9 | 10 | 11 | 12 | 13 | 14 |
| 0 | 323 | 327 | 63.3 | 63.7 | 63.8 | 66.4 | 63.4 | 66.5 | 65.3 | 66.9 | 65.4 | 63.5 | 64.9 | 63.2 |
| 118 | 310 | 327 | 63.6 | 61.4 | 66.9 | 60.9 | 61.7 | 66.9 | 60.9 | 63.2 | 66.4 | 64.4 | 63.8 | 62.6 |
| 174 | 320 | 336 | 65.9 | 63.7 | 62.6 | 59.1 | 63.6 | 68.6 | 61.7 | 64.6 | 64.1 | 66.8 | 65.6 | 63.7 |
| 279 | 301 | 301 | 57.7 | 59.9 | 51.8 | 50.8 | 62.6 | 62.1 | 53.8 | 54.8 | 59.9 | 59.4 | 57.4 | 59.0 |
| | | | | | | | | | | | | | | |

| % Original Activity | | | | | | | | | | | | | | |
|---|---|---|---|---|---|---|---|---|---|---|---|---|---|---|
| | Buffer | | | | | | | | | | | | | |
| Day | B1 | B2 | B3 | B4 | B5 | B6 | B7 | B8 | B9 | B10 | B11 | B12 | B13 | B14 |
| 0 | 100 | 100 | 100 | 100 | 100 | 100 | 100 | 100 | 100 | 100 | 100 | 100 | 100 | 100 |
| 118 | 96.0 | 100.0 | 100.5 | 96.4 | 104.8 | 91.6 | 97.3 | 100.7 | 93.3 | 94.5 | 101.6 | 101.4 | 98.2 | 99.0 |
| 174 | 99.2 | 102.6 | 104.2 | 100.1 | 98.1 | 88.9 | 100.2 | 103.1 | 94.6 | 96.5 | 98.0 | 105.1 | 101.1 | 100.9 |
| 279 | 93.2 | 91.8 | 91.2 | 94.0 | 81.2 | 76.5 | 98.6 | 93.3 | 82.5 | 81.9 | 91.6 | 93.5 | 88.4 | 93.4 |

**TABLE 6**

| **37 C** | | | | | | | | | | | | | | |
|---|---|---|---|---|---|---|---|---|---|---|---|---|---|---|
| Activities by AAPF-pNA Assay in ΔAU min⁻¹ml⁻¹ | | | | | | | | | | | | | | |
| | Buffer | | | | | | | | | | | | | |
| Day | 1 | 2 | 3 | 4 | 5 | 6 | 7 | 8 | 9 | 10 | 11 | 12 | 13 | 14 |
| 0 | 323 | 327 | 63.3 | 63.7 | 63.8 | 66.4 | 63.4 | 66.5 | 65.3 | 66.9 | 65.4 | 63.5 | 64.9 | 63.2 |
| 9 | 313 | 316 | 63.1 | 64.0 | 64.3 | 64.2 | 62.7 | 63.0 | 61.6 | 64.1 | 60.7 | 61.4 | 62.4 | 61.2 |
| 29 | 311 | 320 | 63.3 | 63.2 | 64.1 | 63.4 | 62.3 | 64.4 | 60.4 | 64.3 | 61.1 | 61.7 | 62.7 | 61.4 |
| 112 | 269 | 298 | 57.9 | 55.3 | 62.4 | 48.4 | 64.7 | 63.4 | 48.9 | 62.2 | 60.9 | 58.9 | 57.4 | 56.5 |
| 173 | 281 | 233 | 54.7 | 50.3 | 57.3 | 12.4 | 61.7 | 64.6 | 27.7 | 60.7 | 60.7 | 61.7 | 55.9 | 52.6 |
| 280 | 242 | 43.9 | 48.3 | 44.6 | 6.79 | 4.42 | 56.9 | 58.3 | 4.76 | 32.3 | 52.5 | 55.5 | 47.1 | 42.4 |
| | | | | | | | | | | | | | | |

| % Original Activity | | | | | | | | | | | | | | |
|---|---|---|---|---|---|---|---|---|---|---|---|---|---|---|
| | Buffer | | | | | | | | | | | | | |
| Day | B1 | B2 | B3 | B4 | B5 | B6 | B7 | B8 | B9 | B10 | B11 | B12 | B13 | B14 |
| 0 | 100 | 100 | 100 | 100 | 100 | 100 | 100 | 100 | 100 | 100 | 100 | 100 | 100 | 100 |
| 9 | 97.0 | 96.6 | 99.8 | 100.5 | 100.7 | 96.6 | 98.9 | 94.7 | 94.4 | 95.8 | 92.8 | 96.7 | 96.2 | 96.8 |
| 29 | 96.4 | 97.7 | 100.0 | 99.2 | 100.4 | 95.4 | 98.1 | 96.9 | 92.5 | 96.0 | 93.4 | 97.2 | 96.7 | 97.1 |
| 112 | 83.4 | 90.9 | 91.4 | 86.9 | 97.8 | 72.9 | 102.0 | 95.4 | 75.0 | 93.0 | 93.2 | 92.7 | 88.4 | 89.4 |
| 173 | 87.0 | 71.2 | 86.4 | 79.0 | 89.8 | 18.7 | 97.3 | 97.1 | 42.5 | 90.7 | 92.9 | 97.2 | 86.1 | 83.3 |
| 280 | 75.1 | 13.4 | 76.3 | 70.0 | 10.6 | 6.6 | 89.6 | 87.7 | 7.3 | 48.3 | 80.3 | 87.4 | 72.6 | 67.1 |

### Protease Assay

Examples 1 and 2 measured activity utilizing a protease assay with N-succinyl-L-Ala-L-L-Ala-L-Pro-L-Phe-*p*-nitroanilide (suc-AAPF-pNA). The assay is based upon the cleavage by proteases of the amide bond between phenylalanine and p-nitroaniline of the N-succinyl reagent. P-nitroaniline is monitored spectrophotometrically at 410 nm and the rate of the appearance of *p*-nitroaniline is a measure of proteolytic activity. A protease unit is defined as the amount of protease enzyme that increases absorbance at 410 nm by 1 absorbance unit (AU)/min of a standard solution of 1.6 mM suc-AAPF-pNA in 0.1 M Tris Buffer at 25°C in a cuvet with a 1cm path length. The glycerol was obtained from JT Baker.

## Claims

1. A method for stabilizing one or more protease enzymes from Bacillus species in a liquid medium, consisting of:
formulating in said liquid medium an alkali metal halide salt at a level of at least 4% w/w in combination with a polyol solvent at a level of at least 30% w/w.

2. The method of claim 1, wherein said alkali metal halide salt comprises sodium chloride, said sodium chloride being present at a level of at least 4% w/w.

3. The method of claim 2, wherein said sodium chloride is present at a level of between 5% w/w and 12% w/w.

4. The method of claim 1, wherein said polyol is present at a level of at least 50% w/w.

5. The method of claim 4, wherein said polyol is present at a level of between 50% w/w and 80% w/w.

6. The method of claim 1, wherein said polyol solvent is selected from the group consisting of glycerol, propylene glycol, sucrose, and any combination thereof.

7. The method of claim 1, wherein said one or more protease enzymes is a subtilisin.

8. The method of claim 1, wherein said liquid medium is a liquid enzyme concentrate or a formulated product.

9. The method of claim 8, wherein said liquid medium is a formulated product selected from the group consisting of personal care products, health case products, cleaning products, and detergency products.

10. A stabilized liquid enzyme formulation, consisting of one or more proteases from a Bacillus species, at least 4% w/w of an alkali metal halide salt, and at least 30% w/w of a polyol solvent in a liquid medium.

11. The formulation of claim 10, wherein said alkali metal salt comprises sodium chloride, said sodium chloride being present at a level of at least 4% w/w.

12. The formulation of claim 11, wherein said sodium chloride is present at a level of between 5% w/w and 12% w/w.

13. The formulation of claim 10, wherein said polyol is present at a level of at least 50% w/w.

14. The formulation of claim 13, wherein said polyol is present at a level of between 50% w/w and 80% w/w.

15. The formulation of claim 10, wherein said polyol solvent is selected from the group consisting of glycerol, propylene glycol, sucrose, and any combination thereof.

16. The formulation of claim 10, wherein said one or more protease enzymes includes a subtilisin.

17. The formulation of claim 16, wherein said subtilisin has at least 98% activity remaining after 22 days at 37°C.

18. The formulation of claim 16, wherein said subtilisin includes an amino acid substitution at position 217.

19. The formulation of claim 10, wherein said liquid formulation is a liquid enzyme concentrate or a formulated product.

20. The formulation of claim 19, wherein said liquid formulation is a formulated product selected from the group consisting of personal care products, health care products, cleaning products, and detergency products.

## Patentansprüche

1. Verfahren zum Stabilisieren einer oder mehrerer Proteaseenzyme aus Bacillus species in einem Flüssigmedium, bestehend aus:
Formulieren eines Alkalimetallhalogenidsalzes mit einem Anteil von wenigstens 4% w/w in Kombination mit einem Polyol-Lösungsmittel mit einem Anteil von wenigstens 30% w/w in dem Flüssigmedium.

2. Verfahren nach Anspruch 1, wobei das Alkalimetallhalogenidsalz Natriumchlorid umfasst, wobei das Natriumchlorid mit einem Anteil von wenigstens 4% w/w vorliegt.

3. Verfahren nach Anspruch 2, wobei das Natriumchlorid mit einem Anteil zwischen 5% w/w und 12% w/w vorliegt.

4. Verfahren nach Anspruch 1, wobei das Polyol mit einem Anteil von wenigstens 50% w/w vorliegt.

5. Verfahren nach Anspruch 4, wobei das Polyol mit einem Anteil zwischen 50% w/w und 80% w/w vorliegt.

6. Verfahren nach Anspruch 1, wobei das Polyol-Lösungsmittel aus der Gruppe bestehend aus Glycerin, Propylenglykol, Sucrose und irgendeine Kombination davon ausgewählt ist.

7. Verfahren nach Anspruch 1, wobei das eine oder mehrere Proteaseenzym Subtilisin ist.

8. Verfahren nach Anspruch 1, wobei das Flüssigmedium ein Enzymflüssigkonzentrat oder ein formuliertes Produkt ist.

9. Verfahren nach Anspruch 8, wobei das Flüssigmedium ein aus der Gruppe bestehend aus Körperpflegeprodukten, Körperpflegemitteln, Reinigungsmitteln und Waschmitteln ausgewähltes formuliertes Produkt ist.

10. Stabilisierte Enzymflüssigformulierung, bestehend aus einer oder mehreren Proteasen aus einer Bacillus-Spezies, wenigstens 4% w/w eines Alkalimetallhalogenidsalzes und wenigstens 30% w/w eines Polyol-Lösungsmittels in einem Flüssigmedium.

11. Formulierung nach Anspruch 10, wobei das Alkalimetallsalz Natriumchlorid umfasst, wobei das Natriumchlorid mit einem Anteil von wenigstens 4% w/w vorliegt.

12. Formulierung nach Anspruch 11, wobei das Natriumchlorid mit einem Anteil zwischen 5% w/w und 12% w/w vorliegt.

13. Formulierung nach Anspruch 10, wobei das Polyol mit einem Anteil von wenigstens 50% w/w vorliegt.

14. Formulierung nach Anspruch 13, wobei das Polyol mit einem Anteil zwischen 50% w/w und 80% w/w vorliegt.

15. Formulierung nach Anspruch 10, wobei das Polyol-Lösungsmittel aus der Gruppe bestehend aus Glycerin, Propylenglykol, Sucrose und irgendeine Kombination davon ausgewählt ist.

16. Formulierung nach Anspruch 10, wobei das eine oder mehrere Proteaseenzym ein Subtilisin umfasst.

17. Formulierung nach Anspruch 16, wobei das Subtilisin eine verbleibende Aktivität nach 22 Tagen bei 37°C von wenigstens 98% aufweist.

18. Formulierung nach Anspruch 16, wobei das Subtilisin einen Aminosäureaustausch an Position 217 umfasst.

19. Formulierung nach Anspruch 10, wobei die Flüssigformulierung ein Enzymflüssigkonzentrat oder ein formuliertes Produkt ist.

20. Formulierung nach Anspruch 19, wobei die Flüssigformulierung ein aus der Gruppe bestehend aus Körperpflegeprodukten, Körperpflegemitteln, Reinigungsmitteln und Waschmitteln ausgewähltes formuliertes Produkt ist.

## Revendications

1. Procédé de stabilisation d'une ou de plusieurs enzymes protéases issues de l'espèce Bacillus dans un milieu liquide, consistant à :
formuler dans ledit milieu liquide un sel d'halogénure de métal alcalin à un niveau d'au moins 4 % p/p en mélange avec un solvant polyol à un niveau d'au moins 30 % p/p.

2. Procédé selon la revendication 1, dans lequel ledit sel d'halogénure de métal alcalin comprend du chlorure de sodium, ledit chlorure de sodium étant présent à un niveau d'au moins 4 % p/p.

3. Procédé selon la revendication 2, dans lequel ledit chlorure de sodium est présent à un niveau compris entre 5 % p/p et 12 % p/p.

4. Procédé selon la revendication 1, dans lequel ledit polyol est présent à un niveau d'au moins 50 % p/p.

5. Procédé selon la revendication 4, dans lequel ledit polyol est présent à un niveau compris entre 50 % p/p et 80 % p/p.

6. Procédé selon la revendication 1, dans lequel ledit solvant polyol est choisi dans le groupe constitué du glycérol, du propylène glycol, du sucrose et l'un quelconque de leurs mélanges.

7. Procédé selon la revendication 1, dans lequel ladite ou lesdites enzymes protéases sont une subtilisine.

8. Procédé selon la revendication 1, dans lequel ledit milieu liquide est un concentré enzymatique liquide ou un produit formulé.

9. Procédé selon la revendication 8, dans lequel ledit milieu liquide est un produit formulé choisi dans le groupe constitué des produits d'hygiène personnelle, des produits de soins de santé, des produits de nettoyage et des produits détergents.

10. Formulation enzymatique liquide stabilisée, constituée d'une ou de plusieurs protéases issues d'une espèce Bacillus, d'au moins 4 % p/p d'un sel d'halogénure de métal alcalin et d'au moins 30 % p/p d'un solvant polyol dans un milieu liquide.

11. Formulation selon la revendication 10, dans laquelle ledit sel de métal alcalin comprend du chlorure de sodium, ledit chlorure de sodium étant présent à un niveau d'au moins 4 % p/p.

12. Formulation selon la revendication 11, dans laquelle ledit chlorure de sodium est présent à un niveau compris entre 5 % p/p et 12 % p/p.

13. Formulation selon la revendication 10, dans laquelle ledit polyol est présent à un niveau d'au moins 50 % p/p.

14. Formulation selon la revendication 13, dans laquelle ledit polyol est présent à un niveau compris entre 50 % p/p et 80 % p/p.

15. Formulation selon la revendication 10, dans laquelle ledit solvant polyol est choisi dans le groupe constitué du glycérol, du propylène glycol, du sucrose et de l'un quelconque de leurs mélanges.

16. Formulation selon la revendication 10, dans laquelle ladite ou lesdites enzymes protéases incluent une subtilisine.

17. Formulation selon la revendication 16, dans laquelle ladite subtilisine a au moins 98 % d'activité restante après 22 jours à 37 °C.

18. Formulation selon la revendication 16, dans laquelle ladite subtilisine inclut une substitution d'acide aminé à la position 217.

19. Formulation selon la revendication 10, dans laquelle ladite formulation liquide est un concentré enzymatique liquide ou un produit formulé.

20. Formulation selon la revendication 19, dans laquelle ladite formulation liquide est un produit formulé choisi dans le groupe constitué des produits d'hygiène personnelle, des produits de soins de santé, des produits de nettoyage et des produits détergents.
